# EUROPEAN PATENT SPECIFICATION

(11) **EP 1 319 179 B1**
(45) Date of publication and mention of the grant of the patent: **05.03.2008**
(21) Application number: 01961674.7
(22) Date of filing: 19.07.2001
(51) Int. Cl.: G01N 33/53, C12N 15/09, C12Q 1/68, G01N 37/00

(54) **METHODS FOR DETECTING AND ASSAYING NUCLEIC ACID SEQUENCES**
VERFAHREN ZUR ERKENNUNG UND ZUM ASSAY VON NUKLEINSÄURESEQUENZEN
PROCEDES DE DETECTION ET DE DOSAGE DE SEQUENCES NUCLEOTIDIQUES

(30) Priority: 19.07.2000 US 219397 P
(43) Date of publication of application: 18.06.2003
(60) Divisional application: 06021655.3
(73) Proprietor: Genisphere Inc., Montvale, NJ 07645 (US)
(72) Inventor: GETTS, Robert, C., Collegeville, PA 19426 (US); KADUSHIN, James, M., Gilbertsville, PA 19525 (US)
(74) Representative: Bergstrand, Mikael Gudmundsson
(86) International application number: PCT/US2001/022818
(87) International publication number: WO 2002/006511

(56) References cited:
- WO-A-97/27317
- WO-A-99/20789
- STEARS R L ET AL: "A novel, sensitive detection system for high-density microarrays using dendrimer technology" PHYSIOLOGICAL GENOMICS, AMERICAN PHYSIOLOGICAL SOCIETY, BETHESDA, MD, US, vol. 3, September 2000 (2000-09), pages 93-99, XP002292926 ISSN: 1094-8341
- NILSEN T W ET AL: "DENDRITIC NUCLEIC ACID STRUCTURES" JOURNAL OF THEORETICAL BIOLOGY, ACADEMIC PRESS, LONDON, GB, vol. 187, 1997, pages 273-284, XP002940183 ISSN: 0022-5193
- STEARS ET AL.: 'A novel, sensitive detection system for high-density microarrays using dendrimer technology' PHYSIOL. GENOMICS vol. 3, August 2000, pages 93 - 99, XP002906699
- BEIER ET AL.: 'Versatile derivatisation of solid support media for covalent bonding on DNA-microschips' NUCLEIC ACIDS RESEARCH vol. 27, no. 9, 1999, pages 1970 - 1977, XP002145887
- SHCHEPINOV ET AL.: 'Oligonucleotide dendrimers: synthesis and use as polylabelled DNA probes' NUCLEIC ACIDS RESEARCH vol. 25, no. 22, 1997, pages 4447 - 4454, XP002928476
- KRICKA L. ET AL: 'Nucleic acid detection technologies - Labels, strategies, and formats' CLINICAL CHEMISTRY vol. 45, no. 4, April 1999, US, pages 453 - 458, XP002172731
- GENSCH T. ET AL: 'Fluorescence Detection from Single Dendrimers with Multiple Chromophores', ANGEW. CHEM. INT. ED., vol. 38, no. 24, 1999, pages 3752-3756, DE

## Description

### Field of the Invention

The present invention is related generally to nucleic acid assays, more particularly to methods for detecting and assaying nucleic acid sequences by hybridization.

### Background of the Invention

Changes in gene expression patterns or in a DNA sequence can have profound effects on biological functions. Such variations in gene expression may result in altered physiological and pathological processes. Developing DNA technologies are providing rapid and cost-effective methods for identifying gene expression and genetic variations on a large-scale level. One useful development is the DNA microarray useful for rapidly detecting and assaying samples of nucleic acid sequences. Each microarray is capable of performing the equivalent of thousands of individual "test tube" experiments over a short time period thereby providing rapid and simultaneous detection of thousands of expressed genes. Microarrays have been implemented in a range of applications such as analyzing a sample for the presence of gene variations or mutations (i.e. genotyping), or for patterns of gene expression.

Generally, a microarray comprises a substantially planar substrate such as a glass cover slide, a silicon plate or nylon membrane, coated with a grid of tiny spots or features of about 20 microns or smaller in diameter. Each spot or feature contains millions of copies of a specific sequence of nucleic acid extracted from a strand of deoxyribonucleic acid (DNA). Due to the number of features involved, a computer is typically used to keep track of each sequence located at each predetermined feature. Messenger RNA (mRNA) is extracted from a sample of cells. The mRNA serving as a template, is reverse transcribed to yield complementary DNA (cDNA). As a first example of the prior art techniques, one or more labels or markers such as fluorescence are directly incorporated into the copies of cDNA during the reverse transcription process. The labeled copies of cDNA are broken up into short fragments and washed over the microarray. Under suitable hybridization conditions, the labeled fragments are hybridized or annealed with complementary nucleic acid sequences (i.e. gene probes) attached to the features of the microarray for ready detection thereof. This labeling method has been commonly referred to as "direct incorporation".

Upon hybridization of the cDNA to the microarray, a detectable signal (e.g. fluorescence) is emitted for a positive outcome from each feature containing a cDNA fragment hybridized with a complementary nucleic acid sequence attached thereto. The detectable signal is visible to an appropriate sensor device or microscope, and may then be detected by the computer or user to generate a hybridization pattern. Since the nucleic acid sequence at each feature is known, any positive outcome (i.e. signal generation) at a particular feature indicates the presence of the complementary cDNA sequence in the sample cell. Although there are occasional mismatches, the attachment of millions of gene probes at each spot or feature ensures that the detectable signal is strongly emitted only if the complementary cDNA of the test sample is present.

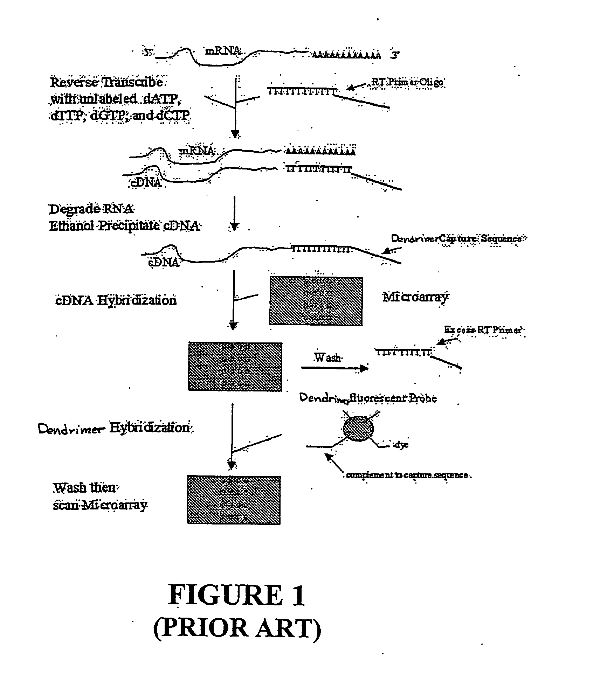

A second example of a prior art method of preparing a nucleic acid sequence sample for detection and assay by a microarray is shown in Figure 1 and described as follows. Using known methods, a plurality of gene probes consisting of known nucleic acid sequences are each affixed or printed at a predetermined location on the surface of a microarray. The attachment of the gene probe to the microarray is typically accomplished through known robotic or laser lithographic processes. The sample is extracted from cells in the form of RNA. Since RNA is relatively unstable and decomposes rapidly and easily, a more stable and resistant form of nucleic acid is typically used. The stable nucleic acid is complementary DNA which is prepared from the RNA sample (e.g. total RNA and poly(A)+ RNA) through known techniques of reverse transcription. Reverse transcription primers (RT primers) having a capture sequence attached thereto, is used in reverse transcription process. As a result, the target cDNA is formed with the capture sequence located at the 5' end. The newly formed target cDNA with the capture sequence is then isolated from the mRNA sample and precipitated. The target cDNA is hybridized to the complementary gene probes affixed to the microarray. After the target cDNA and the microarray are hybridized, the microarray is washed to remove any excess RT primers prior to labeling. A mixture containing labeled "dendritic nucleic acid molecules", or "dendrimers", is then prepared.

Dendrimers are complex, highly branched molecules, and are comprised of a plurality of interconnected natural or synthetic monomeric subunits of double-stranded DNA forming into stable spherical-like core structures with a predetermined number of "free ends" or "arms" extending therefrom. Dendrimers provide efficient means for labeling reactions such as fluorescence, for example, and facilitate direct calculations of the number of transcripts bound due to their predetermined signal generation intensity and proportional relationship to the bound cDNA on the microarray.

Each dendrimer includes two kinds of hybridization "free ends" or "arms" extending from the core surface. Each dendrimer may be configured to include at least one hundred arms of each kind. The arms are each composed of a single-stranded DNA of a specific sequence that can be ligated or hybridized to a functional molecule such as a target or a label. The target molecule can be attached to one kind of arm to provide the dendrimer with targeting capabilities, and the label molecule can be attached to the other kind of arm to provide the dendrimer with signal emission capabilities for detection thereof. The targeting molecule is typically an oligonucleotide that is complementary to the capture sequence of a target, and the label molecule is typically an oligonucleotide linked to a label or marker. Using simple DNA labeling, hybridization, and ligation reactions, a dendrimer may be configured to act as a highly labeled, target specific probe. Dendrimers are described in greater detail in Nilsen et al., Dendritic Nucleic Acid Structures, J. Theor. Biol., 187, 273-284 (1997); and Stears et al., A Novel, Sensitive Detection System for High-Density Microarrays Using Dendrimer Technology, Physiol. Genomics, 3: 93-99 (2000), the entire content of each are incorporated herein by reference.

The prepared mixture is formulated in the presence of a suitable buffer to yield a dendrimer hybridization mixture containing dendrimers with labels attached to one kind of arm, and with oligonucleotides complementary to the capture sequences of the cDNA attached to the other type of arm. The labeled dendrimers are added to the microarray for hybridization with the capture sequences of the bound cDNA to generate a detectable signal from the corresponding feature. The microarray is washed to remove any excess unhybridized dendrimer molecules to reduce unwanted noise generation. The microarray is scanned using conventional techniques to detect the detectable signal emitted by the labels to generate a particular hybridization pattern for analysis. It is known that the above-described prior art methods require significant time, effort and labor in the preparation and assay of the sample including the hybridization and washing steps.

It would be highly desirable to substantially reduce the amount of time and the number of steps required for preparing a sample and performing the assay without sacrificing desirable attributes such as sensitivity, low background "noise", and minimal "false positives". It would be a significant advance in gene expression detection microarrays to further provide a method that significantly reduces the complexity and the labor needed to prepare the gene samples and does not require RNA extracted directly from a sample to be converted to cDNA prior to hybridization, but rather uses the RNA sample directly in the methods of the present invention for gene expression analysis, and which can be carried out using conventional laboratory reagents, equipment and techniques.

### Summary of the Invention

The present invention relates generally to methods for assaying and detecting the presence of a specific sequence of nucleotides in a nucleic acid target molecule of a sample through the process of hybridization and does not require the time- and labor- consuming enzymatic conversion of that sample into a complementary copy. The present invention significantly reduces the time and labor that are typically required to process and assay the nucleic acid target molecule for obtaining information about the genetic profile of the target nucleic acid sample and the source from which the sample was obtained. The present invention further provides a microarray with excellent sensitivity and low background "noise", and minimal "false positives". The method of the present invention may be used in a range of genomic applications such as gene expression profiling and high-throughput functional genomic analysis.

The present invention is directed to method for determining the presence of a specific nucleotide sequence in an RNA reagent of a target sample. In a particular aspect of the invention, the method comprises the steps of:
a) incubating a mixture comprising:
   (i) a first component including an RNA reagent extracted directly from a target sample, said RNA reagent having a target nucleotide sequence and a capture sequence, and
   (ii) a second component comprising a capture reagent having at least one first arm containing a label capable of emitting a detectable signal and at least one second arm having a nucleotide sequence complementary to the capture sequence of the RNA reagent of the first component,
      at a first temperature and for a time sufficient to induce the capture sequence of the RNA reagent of the first component to bind to the complementary nucleotide sequence of the capture reagent of the second component, and thereby form a pre-hybridized RNA-capture reagent complex comprising the target nucleotide sequence;
b) contacting the pre-hybridized RNA-capture reagent complex with a microarray having thereon a plurality of features each containing a particular probe nucleotide sequence; and
c) incubating the pre-hybridized RNA-capture reagent complex on the microarray at a second temperature and for a time sufficient to hybridize the target nucleotide sequence of the pre-hybridized RNA-capture reagent complex to the complementary probe nucleotide sequence contained within the feature, wherein the presence of such hybridization results in the emission of the detectable signal from the corresponding feature, and the absence thereof results in no emission of the detectable signal from the corresponding feature, thus generating a detectable hybridization pattern for subsequent analysis.

In another aspect of the present invention, there is provided a method for determining the presence of a specific nucleotide sequence in an RNA reagent of a target sample, the method comprising the steps of:
a) contacting a first component including an RNA reagent extracted directly from a target sample, said RNA reagent having a target nucleotide sequence and a capture sequence with a microarray having thereon a plurality of features each containing a particular probe nucleotide sequence;
b) incubating the RNA reagent and the complementary probe nucleotide sequences on the microarray at a first temperature and for a time sufficient to hybridize the target nucleotide sequence of the RNA reagent to the complementary probe nucleotide sequence contained within the feature;
c) contacting a second component comprising a capture reagent having at least one first arm containing a label capable of emitting a detectable signal and at least one second arm having a nucleotide sequence complementary to the capture sequence of the RNA reagent of the first component; and
d) incubating the capture reagent and the capture sequence of the RNA reagent at a second temperature and for a time sufficient to induce the capture sequence of the RNA reagent of the first component to hybridize to the complementary nucleotide sequence of the capture reagent of the second component, wherein the presence of the hybridization results in the emission of the detectable signal from the corresponding feature, and the absence thereof results in no emission of the detectable signal from the corresponding feature, thus generating a detectable hybridization pattern for subsequent analysis.

In a preferred form of the invention the preferred capture reagent is a dendrimer.

### Brief Description of the Drawings

The following drawings in which like reference characters indicate like parts are illustrative of embodiments of the invention and are not to be construed as limiting the invention as encompassed by the claims forming part of the application.

Figure 1 is a schematic representation of prior art steps for preparing and assaying a target nucleic acid sample on a microarray; and

Figure 2 is a schematic representation of a method for preparing and assaying a target nucleic acid sample on a microarray in one embodiment of the present invention.

### Detailed Description of the Invention

The present invention is generally directed to a method for preparing a nucleic acid sequence sample for detection and assay on a microarray in a manner that provides a significant reduction in time and effort typically required in assaying a genomic sample on a microarray. The method of the present invention provides the advantage of preparing the nucleic acid sequence sample in shorter period of time, using fewer steps but providing the sensitivity, low background "noise", and minimal "false positives" required for laboratory and clinical use. The cost effective and efficient manner by which the nucleic acid sequence samples are prepared and by which the method of the present invention can be implemented using conventional laboratory techniques, equipment and reagents, makes the present invention especially suitable for use in genomic applications such as gene expression profiling and high-throughput functional genomic analysis.

Before the present invention is further described, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

In the methods of the present invention, an array of DNA or gene probes fixed or stably associated with the surface of a substantially planar substrate is prepared as conventionally known in the art. A variety of different microarrays that may be used are known in the art. The substrates with which the gene probes are stably associated may be fabricated from a variety of materials, including plastic, ceramic, metal, gel, membrane, glass, and the like. The microarrays may be produced according to any convenient methodology, such as pre-forming the gene probes and then stably associating them with the surface of the support or growing the gene probes directly on the support. A number of different microarray configurations and methods for their production are known to those of skill in the art, as described, for example, in Science, 283, 83, 1999, the content of which is incorporated herein by reference.

The DNA or gene probes of the microarrays which are capable of sequence specific hybridization with a target nucleic acid sequence extracted from a target sample of cells, may be comprised of polynucleotides or hybridizing analogues or mimetics thereof, including, but not limited to, nucleic acid in which the phosphodiester linkage has been replaced with a substitute linkage group, such as phosphorothioate, methylimino, methylphosphonate, phosphoramidate, guanidine and the like, nucleic acid in which the ribose subunit has been substituted, e.g. hexose phosphodiester; peptide nucleic acid, and the like. The length of the probes will generally range from 10 to 1000 nucleotides. In the preferred embodiment, the DNA or gene probes are each arranged or sequenced for hybridization with the target nucleic acid sequence comprising RNA, more preferably mRNA from the gene of concern.

In some embodiments of the invention, the probes will be oligonucleotides having from 15 to 150 nucleotides and more usually from 15 to 100 nucleotides. In other embodiments the probes will be longer, usually ranging in length from 150 to 1000 nucleotides, where the polynucleotide probes may be single or double stranded, usually single stranded, and may be PCR fragments amplified from cDNA, cloned genes; or other suitable sources of nucleic acid sequences. The DNA or gene probes on the surface of the substrates will preferably correspond to, but are not limited to, known genes of the physiological source being analyzed and be positioned on the microarray at a known location so that positive hybridization events may be correlated to expression of a particular gene in the physiological source from which the target nucleic acid sample is derived. Because of the manner in which the target nucleic acid sample is generated, as described below, the microarrays of gene probes will generally have sequences that are complementary to the RNA strands, including but not limited to, messenger RNA strands, of the gene to which they correspond.

The term "label" is used herein in a broad sense to refer to agents that are capable of providing a detectable signal, either directly or through interaction with one or more additional members of a signal producing system. Labels that are directly detectable and may find use in the present invention include, for example, fluorescent labels such as fluorescein, rhodamine, BODIPY, cyanine dyes (e.g. from Amersham Pharmacia), Alexa dyes (e.g. from Molecular Probes, Inc.), fluorescent dye phosphoramidites, and the like; and radioactive isotopes, such as ³²S, ³²P, ³H, etc.; and the like. Examples of labels that provide a detectable signal through interaction with one or more additional members of a signal producing system include capture moieties that specifically bind to complementary binding pair members, where the complementary binding pair members comprise a directly detectable label moiety, such as a fluorescent moiety as described above. The label is one that preferably does not provide a variable signal, but instead provides a constant and reproducible signal over a given period of time.

The present invention further utilizes a capture reagent which is composed of at least one first arm containing a label capable of emitting a detectable signal and at least one second arm having a nucleotide sequence complementary to a capture sequence attached to the target nucleic acid such as RNA, for example. One such example is a "dendritic nucleic acid molecule", or "dendrimer". Briefly, dendrimers are complex, highly branched molecules, and are comprised of a plurality of interconnected natural or synthetic monomeric subunits of double-stranded DNA forming into stable, spherical-like core structures with a pre-determined number of "arms" or "free ends" extending therefrom for the purposes descried herein. Typically, the capture reagent will have multiple, typically many first and second arms. Besides dendrimers, carbohydrates, proteins, nucleic acids, and the like may be used as the capture reagent. Dendrimers will be described hereinafter as illustrative of suitable capture reagents.

Each dendrimer may be configured to include two kinds of hybridization "free ends" or "arms" extending from the core surface. Each dendrimer may be configured to include at least one hundred arms of each kind. The arms are each composed of a single-stranded DNA of a specific sequence that can be ligated or hybridized to a functional molecule such as a targeting molecule or a label. The targeting molecule can be attached to one kind of arm to provide the dendrimer with targeting capabilities, and the label molecule can be attached to the other kind of arm to provide the dendrimer with signal generation capabilities for detection. The targeting molecule is typically an oligonucleotide that is complementary to the target nucleic acid sequence, and the label molecule is typically an oligonucleotide linked to a label or marker. Using simple DNA labeling, hybridization, and ligation reactions, a dendrimer may be configured to act as a highly labeled, target specific probe molecule.

Further information regarding the structure, configuration and production of dendrimers is also disclosed in U.S. Pat. Nos. 5,175,270, 5,484,904, and 5,487,973, the contents of each are incorporated herein by reference.

In accordance with one embodiment of the present invention, a target nucleic acid, preferably in the form of RNA, and extracted directly from a sample, is contacted with the DNA microarray under conditions sufficient to induce hybridization therebetween, resulting in a hybridization pattern of complementary gene probe/target complexes. Prior to or following the hybridization of the target nucleic acid and the microarray, labeled capture reagents (e.g. dendrimers) under suitable conditions are bonded to the target nucleic acid, whereupon a detectable signal may generated to subsequently render the particular hybridization pattern visible. It is noted that the hybridizations between the target nucleic acids and the microarray, and between the target nucleic acid and the capture reagent (e.g. dendrimer) may be carried out in any suitable order. The labeled capture reagents (e.g. dendrimers) are each capable of generating the same signal of known intensity, thus each positive signal in the microarray can be "counted" in order to obtain quantitative information about the genetic profile of the target nucleic acid sample.

In one embodiment of the present invention, fluorescent labeled dendrimers may be prepared by ligating a nucleic acid sequence or strand complementary to the capture sequence of a target nucleic acid, preferably polydeoxyribothymidine (poly dT), to the purified dendritic core material as prepared by the previously described methods (see Nilson et al., and Stears et al., supra; and the '270, '904, and '973 patent citations mentioned previously). Labeled dendrimers ligated with the poly dT, are able to target and hybridize with a target nucleic acid such as polyadenylated RNA (poly(A)+ RNA), having a capture sequence composed of polydeoxyriboadenidine (poly dA).

As mentioned above, the preferred target nucleic acid is RNA. RNA has rarely been used directly as target nucleic acid samples in the prior art genomic assays due to poor performance results. RNA has an undesirable tendency to decompose rapidly and easily and conventional labeling methods fail to generate adequate signal when used in conjunction with RNA. By utilizing capture reagents (e.g. dendrimers) and the methods of the present invention, the amount of time and the number of steps required to carry out the genomic assays is reduced, thus enabling the use of RNA at least substantially without the degree of RNA degradation and decomposition typically associated with prior art genomic assays. Furthermore, the direct use of RNA precludes the need to reverse transcribe the RNA sample to yield a target cDNA for further reduction in time and effort in carrying out an assay.

The use of RNA also permits microarrays of the present invention to be reused simply by washing them at a suitable temperature with a weak base solution comprising, for example, 0.05 M sodium hydroxide. The base solution denatures and purges the hybridized target RNA from the microarray without damaging the DNA probes or leaving any nucleic residue that can generate background noise in subsequent assays. The preferred range for the washing temperature is from about 50° to 60°C.

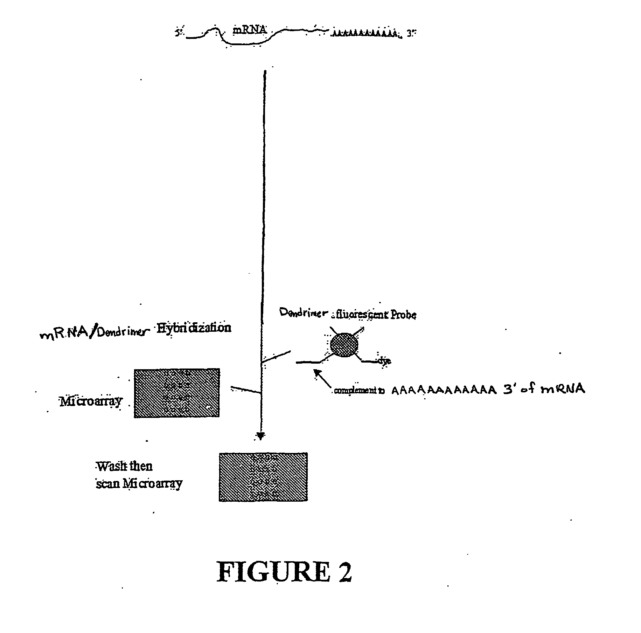

With reference to Figure 2, the method for one embodiment of the present invention generally comprises hybridizing an RNA target sample having a polyadenylated 3' end to a complementary 5'-dendrimer poly dT capture sequence to form a pre-hybridized RNA/dendrimer complex. The pre-hybridized RNA/dendrimer complex subsequently contacts the microarray, comprising multiple features each containing a specific nucleic acid sequence, typically in the form of a cDNA fragment. The RNA molecule hybridizes with the complementary cDNA at a particular feature. The microarray is washed to purge any unhybridized dendrimers that may obscure the signal detection of the hybridized RNA/cDNA and the resulting hybridization pattern. The hybridized RNA/cDNA microarray is then scanned using any suitable commercially available microarray scanner.

In the present invention, the target nucleic acid generally comprising an RNA derived from a naturally occurring source, the RNA may be selected from total RNA, poly(A)⁺ RNA, amplified RNA and the like. The initial RNA source may be present in a variety of different samples, where the sample will typically be derived from a physiological source. The physiological source may be derived from a variety of eukaryotic sources, with physiological sources of interest including sources derived from single celled organisms such as yeast and multi-cellular organisms, including plants and animals, particularly mammals, where the physiological sources from multi-cellular organisms may be derived from particular organs or tissues of the multi-cellular organism, or from isolated cells derived therefrom.

In obtaining the sample RNAs to be analyzed, the physiological source may be subjected to a number of different processing steps, where such known processing steps may include tissue homogenation, cell isolation and cytoplasmic extraction, nucleic acid extraction and the like. Methods of isolating RNA from cells, tissues, organs or whole organisms are known to those of ordinary skill in the art and are described, for example, in Maniatis et al., Molecular Cloning: A Laboratory Manual, 2nd ed., Cold Spring Harbor Laboratory Press, 1989, and in Ausubel et al., Current Protocols in Molecular Biology, John Wiley & Sons, Inc., 1998, the content of each are incorporated herein by reference.

More preferably, the target nucleic acid is a polyadenylated RNA (poly(A)+ RNA). The poly(A)+ RNA includes an oligonucleotide which is comprised of a strand of adenine bases, or poly dA sequence, and provides a hybridization site for a dendrimer having a complementary oligonucleotide which is comprised of a strand of thymine bases, or poly dT sequence. By utilizing a poly(A)+ RNA and a dendrimer with a poly dT sequence, an RNA/dendrimer complex can be readily formed under suitable hybridization conditions. The RNA then binds to a complementary cDNA on the microarray under suitable hybridization conditions.

It is noted that poly(A)+ RNA is typically present in most genomic samples and in all genomic samples of mammalian origin such as from humans, mice, rats, pigs and the like. The present invention may also be used in conjunction with non-poly(A)+ RNA samples as well. Such non-poly(A)+ RNA lacks a poly dA sequence useful as a capture sequence in the present invention. Accordingly, such non-poly(A)+ RNA is prepared by attaching or ligating a capture sequence prior to contact with the dendrimer or the microarray.

In one embodiment of the present invention, a suitable capture sequence may be ligated to the RNA using conventional methods of splicing RNA such as through enzymatic means. Such suitable capture sequences may include poly dA sequences or customized nucleic acid sequences that are attached through methods known to those of ordinary skill in the art. Typically, a ligase enzyme can be suitably employed to splice two oligonucleotides or RNA strands to one another, or covalently couple a synthetic sequence to the end of the RNA. In the alternative, if the RNA includes a specific oligonucleotide that is useful as a capture sequence, a complementary oligonucleotide may be attached to the dendrimer for carrying out the goals of the present invention. In a preferred embodiment, the non-poly(A)+ RNA is ligated to a poly dA sequence through the use of an enzyme capable of forming an oligonucleotide of adenine bases onto the RNA strand. Such an enzyme may include poly-ADP-ribose polymerase. Either of these methods is particularly desirable in the event that the RNA sample of choice does not have an appropriate capture sequence required to carry out the methods of the present invention. The enzymatic processes as mentioned above can readily be performed by those of ordinary skill in the art.

In the present invention, the capture sequence of the present invention may be further adapted to include modified nucleotides comprising locked nucleic acids (LNA) for increasing the nucleotide melt or annealing temperature attributes of the corresponding annealed capture sequences to above that normally associated with standard unmodified sequences. During hybridization process, there is the prospect that the bonding between the capture sequences of the dendrimer and the RNA may be fragile due to low melt temperature attributes especially when hybridized at higher than optimal temperatures. By including such modified higher melt temperature nucleotides, a stronger annealing bond is formed for a durable and permanent hybridization between complementary nucleotides. Oligonucleotides containing LNA modified nucleotides obey the Watson-Crick base pairing rules and hybridize to complementary oligonucleotides. However, LNA oligonucleotides demonstrate an improved hybridization performance. LNA/DNA and LNA/RNA duplexes are more thermally stable and demonstrate a 3° to 8°C increase in thermal stability per modified base. Locked nucleic acids are a novel class of nucleic acid analogues that have the furanose ring of the nucleotide modified to contain methylene linker that connects the 2'-oxygen position to the 4'-carbon position thus altering the DNA backbone and stabilizing nucleic acid duplexes. Such LNA modified nucleotides may include, but not limited to, peptide nucleic acids and the like. Synthetic oligonucleotides containing LNA nucleotides are available from PROLOGO (Boulder, CO). In one embodiment of this invention, the poly dT sequence may be synthesized to contained LNA (Locked Nucleic Acid) thymidine nucleotides (LNA-T) at several positions. In this embodiment dendrimer reagents prepared using LNA modified poly dT would have the beneficial effect of increase thermal stability of hybridization between the poly A capture sequence of the RNA and the locked nucleic acid comprising thymine base (LNA-T) sequence on the labeled dendrimers preferably preventing the exchange of dendrimers from one RNA target to another.

In a preferred embodiment of the present invention, the dendrimers are hybridized with a poly (A)+ RNA under suitable conditions, and allowed to bind to the 5' end of the RNA via a capture sequence comprising an oligonucleotide of poly dT units. The RNA/dendrimer complexes are then hybridized to the microarray between the RNA and the complementary cDNA located at a particular feature, and the unbound dendrimers are then washed off. The dendrimers pre-labeled with a cyanine dye such as Cy3 or Cy5 are sensitive detection reagents especially suitable for high-density DNA microarrays.

Once the RNA/dendrimer complexes are formed, blocking nucleic acids or reagents including, but not limited to, dA and/or dT oligonucleotides, may be added as blocking nucleic acids or reagents to prevent unwanted hybridization which may generate "false positives". Such blocking nucleic acids are comprised of short segments complementary to the capture sequences on the dendrimers and the target RNA, respectively. The addition of blocking nucleic acids specifically prevents binding of excess dendrimers to certain features on the microarray that may contain short segments of poly dA or sequences complementary to the capture sequence on the dendrimer that may undesirably generate non-specific hybridizations. It is further desirable to add the blocking nucleic acids to the pre-hybridized RNA/dendrimer, thus preventing any exchange of dendrimers from one target RNA to another. Such blocking nucleic acids may range from about 15 to 200 base units. A preferred amount of the blocking nucleic acid may range from about 0.1 to 10 micrograms to every microgram of RNA sample used for assay. It may also be desirable to add an amount of yeast tRNA in order help protect the RNA from degradation during subsequent hybridization of the RNA to the microarray.

In order to maintain the target RNA in an intact and stable condition suitable for detection and assay, it is further preferable to add a nuclease inhibitor more preferably a ribonuclease inhibitor to protect the target RNA during the hybridization steps. An RNA preserving solution such as RNA SECURE® (available from AMBION, Austin, TX) may also be added to the target RNA for substantially reducing the rate of decomposition and degradation of the target RNA.

In performing the methods of the present invention, a quantity of poly(A)+ RNA with a polydA capture sequence, is hybridized with dendrimers with an oligonucleotide complementary to the RNA capture sequence (i.e. poly dT). The capture sequences and the complement have sufficient base units to irreversibly hybridize under suitable conditions including time and temperature sufficient for promoting the hybridization of the dendrimer to the target RNA as known by those of ordinary skill in the art. Suitable hybridization conditions are disclosed in Maniatis et al., where conditions may be modulated to achieve a desired specificity in hybridization. It is further noted that any suitable hybridization buffers may be used in the present invention. The preferred range for the first hybridization temperature is from about 45° to 60°C, more preferably 55°C. The mixture is incubated for a time from about 15 minutes to 24 hours, preferably from about 15 minutes to 16 hours, more preferably from about 15 to 20 minutes to allow the target RNA to hybridize with the dendrimers to yield a prehybridization mixture.

The pre-hybridization mixture is added to the microarray and incubated at a second hybridization temperature and for a time sufficient to permit the RNA to bind to the microarray. The prehybridization mixture and the microarray are incubated at the second hybridization temperature from about 15 minutes to 24 hours, preferably from about 2 to 16 hours in a humidified chamber. The preferred range for the second hybridization temperature is from about 60° to 65°C for microarrays prepared with gene probes derived from cDNAs, and from about 45° to 55°C for microarrays prepared from gene probes derived from oligonucleotides. The temperatures provided above may be adjusted in order to suit the requirements necessary to ensure complete hybridization to a selected microarray and can be suitably determined by those of ordinary skill in the art.

Following the hybridization step, where non-hybridized dendrimers are capable of emitting a signal during the detection step, a washing step is employed to purge the non-hybridized complexes from the microarray, thus leaving behind a visible, discrete pattern of hybridized RNA-dendrimers bound to the microarray. A variety of wash solutions and protocols for their use are known to those of skill in the art and may be used. The specific wash conditions employed will necessarily depend on the specific nature of the signal producing system that is employed, and will be known to those of skill in the art familiar with the particular signal producing system employed.

The resultant hybridization pattern of labeled RNA fragments may be visualized or detected in a variety of ways, with the particular manner of detection being chosen based on the particular label of the dendrimer used in the present invention, where representative detection systems include scintillation counting, autoradiography, fluorescence measurement, calorimetric measurement, light emission measurement and the like.

Following hybridization and any washing step(s) and/or subsequent treatments, as described above, the resultant hybridization pattern is detected. In detecting or visualizing the hybridization pattern, the intensity or signal value of the label may be qualitatively and/or quantitatively detected.

Following detection or visualization, the hybridization pattern can be used to determine qualitative and/or quantitative information about the genetic profile of the labeled target nucleic acid sample that was contacted with the microarray to generate the hybridization pattern, as well as the physiological source from which the labeled target nucleic acid sample was derived. From this data, one can also derive information such as the types of genes expressed in the tissue or cell that is the physiological source, as well as the levels of expression of each gene. Where one uses the subject methods in comparing target nucleic acid from two or more physiological sources, the hybridization patterns may be compared to identify differences between the patterns. With microarrays in which each of the different probes corresponds to a known gene is employed, any discrepancies can be related to a differential expression of a particular gene in the physiological sources being compared. Thus, the subject methods find use in differential gene expression assays, where one may use the subject methods in the differential expression analysis of: diseased and normal tissue, e.g. neoplastic and normal tissue; different tissue or subtissue types; and the like.

The forgoing discussion discloses and describes merely exemplary embodiments of the present invention. One skilled in the art will readily recognize from such discussion, and from the accompanying claims, that various changes, modifications, and variations can be made therein without departing from the spirit and scope of the invention as defined in the following claims.

### EXAMPLE 1

A method for determining the presence of a specific sequence of nucleotides in a nucleic acid target molecule sample on a microarray is described below.

### Microarray Preparation

The microarray was prepared by spotting 1,700 amplified cDNAs on a sialylated glass slide (Cell Associates) using a microarrayer (Cartesian Technologies), after which the cDNA was ultraviolet (UV) cross-linked onto the slide surface with a Stratalinker (Stratagene).

### Preparation and Concentration of Target Nucleic

### Acid Sequences Sample

The target nucleic acid sequences were prepared as total RNA extracted from a sample of cells using standard methods. For this particular example the portion of the total RNA population comprising that to be know as poly(A)+ RNA also commonly referred to as messenger RNA (mRNA) was used as the specific target sample. The sequence comprising an oligonucleotide of single adenine bases of the RNA served as the desired capture sequence.

### Preparation of the Labeled Dendrimers

Dendrimers were assembled from seven oligonucleotides, strands 1-7. The seven strands were pairwise hybridized in T₅₀ N₂₀₀ E₁₀ 8 buffer (T₅₀ N₂₀₀ E₁₀ 8: 50 mM Tris-HCl, pH 8.0, 10 mM EDTA, and 200 mM NaCl) to form five building block "monomers": A, B',B",C', and C". Strand 1 and strand 2 were hybridized to form the "A" monomer. Similarly, strand 3 was hybridized to strand 4 and strand 4 was hybridized to strand 5 to produce the B' and B" monomers, respectively. The C' monomer was prepared by hybridizing strand 2 and strand 6, and the C" monomer was prepared by hybridizing strand 2 and strand 7. Each monomer is composed of four 31-nucleotide single-stranded arms and one central 50-base waist. From these building blocks the core dendritic structure was assembled.

Dendrimer core assembly was accomplished by hybridizing one initiating A monomer with two B' and two B" monomers in T₅₀ N₂₀₀ E₁₀ 8 buffer for 20 min at 42°C to form the 1-layer dendrimer. A second layer of C' and C" monomers was added to the one-layer assembly to form a two-layer dendrimer regenerating the original sequences present on the A monomer. Three- and four-layer dendrimers were prepared by the sequential addition of B' and B" monomers followed by C' and C" monomers. At each layer of assembly 4,5',8-trimethylpsoralen (trioxsalen) was used to covalently cross-link the dendritic structure. This was accomplished by adding 1/15 of a volume of a trioxsalen-saturated ethanol solution to the hybridization mixture.

After 20 min of incubation, the hybridization mixture was cross-linked in a UV photochemical reaction chamber (model HRI100, Simms Instruments) for 10 min at 42°C. After the forth layer of assembly, the four-layer dendritic core was purified using ultracentrifugation on 10-50% sucrose gradients containing 50% deionized formamide run at 40°C. Gradient fractions containing four-layer dendrimer were pooled, ethanol precipitated, and resuspended in 50 mM Tris-HCl, pH 8.0, 10 mM EDTA.

To prepare fluorescent-labeled dendrimers, a synthetic dT70 sequence was ligated to the purified dendritic core material. Thirty-nucleotide-long oligonucleotides complementary to the outer arms of the four-layer dendrimer, and having a Cy3-, Cy5-, or other equivalent desirable label at the 5' end, were synthesized (Oligos Etc.). In one embodiment for single channel analysis dT70 containing core material was placed into one separate container and Cy3-linked oligonucleotides were hybridized and covalently cross-linked to the outer surface of the dendrimer material. In a second embodiment for dual channel analysis the dT70 containing core material was split into two separate containers/populations. The Cy3-linked and Cy5-linked oligonucleotides were hybridized and covalently cross-linked to the outer surface of the two dendrimer populations, separately thus producing one dendrimer reagent having an oligo dT70 associated with Cy3 label and a second separate reagent having an oligo dT70 associated with a Cy5 label. In a third embodiment for three or more channel analysis the dT70 containing core material was split into the respective number of containers/populations. As in the dual channel reagents labeled oligonucleotides were added to the appropriate population, hybridized and cross-linked as separate reagents thus producing three or more different reagents each being unique as defined by the associated label.

Any excess capture and fluorescent oligonucleotides were removed by size exclusion chromatography. The concentration of dendrimer was determined by measuring the optical density of the purified material at 260 nm on a spectrophotometer. The fluorescence was measured at the optimal signal/noise wavelengths using a fluorometer (FluoroMax, SPEX Industries). Cy3 was excited at 542 nm, and the emission was measured at 570 nm. Cy5 was excited at 641 nm, and the emission was measured at 676 nm.

### Hybridization of RNA to Labeled Dendrimers

An RNA hybridization buffer was prepared with the following formulation: 4.5% sodium dodecyl sulfate, 200 mM sodium chloride, and 0.1M sodium phosphate. The RNA hybridization buffer was thawed and resuspended by heating to 65°C for ten to twelve minutes. The buffer was mixed by inversion to ensure that the components were re-suspended evenly. The heating and mixing was repeated until all of the material was re-suspended. The RNA was resuspended in 5.0 µL of the hybridization buffer.

In a first embodiment, single channel analysis, 2.5 µl of one type of the labeled dendrimers (Cy3 or Cy5) were added to the re-suspended RNA along with 12.5 µL of the RNA hybridization buffer. In an alternative embodiment, for dual channel analysis, 2.5 *µ*l of two types of the labeled dendrimers with Cy3 and Cy5, respectively, were added to the re-suspended RNA along with 10 *µ*L of the RNA hybridization buffer. In a further embodiment of multiple channel analysis (with three or more channels), 2.5 *µ*l of three or more types of the labeled dendrimers comprising Cy3, Cy5, and one or more prepared using another label moiety, respectively, were added to the re-suspended RNA along with 10 µL of the RNA hybridization buffer.

For larger hybridization buffer volumes, additional RNA hybridization buffer may be added to the required final volume. It is noted that hybridization buffer volumes greater than 35 *µ*L may also require additional amounts of the labeled dendrimers.

The RNA hybridization buffer mixture was incubated at 55°C for 15 to 20 minutes to allow for pre-hybridization of the RNA to the labeled dendrimers. In one embodiment where dual or multiple channel analysis was implemented, an amount of about 2 micrograms of oligo dT70 was added to the pre-hybridized RNA/dendrimer mixtures to prevent the exchange of dendrimers between the RNA.

The pre-hybridized mixture was added to the microarray and then incubated from 2 hours to overnight at 55°C in a humidified chamber at a temperature from about 60° to 65°C for microarrays prepared from cDNA and from about 45° to 55°C for microarrays prepared from oligonucleotides. At this stage, the RNA was hybridized to the gene probes.

### Post Hybridization Wash

The microarray was briefly washed to remove any excess labeled dendrimers. First, the microarray was washed for 10 minutes at 55° C with 2X SSC buffer, 0.2% SDS. Then the microarray was washed for 10 minutes at room temperature with 2X SSC buffer. Finally the microarray was washed for 10 minutes at room temperature with 0.2X SSC buffer.

### Signal Detection

The microarray was then scanned using a confocal laser scanner (ScanArray 3000 available from GSI Lumonics). The signal and background signals were detected and quantified using Imagene quantification software. Signals were measured as the means pixel intensity within each circumscribed feature, average background was measured using the mean pixel intensities of blank features (no gene probe) in the microarray and as the means pixel intensity outside the circumscribed feature diameter for calculating specific signals.

## Claims

1. A method for determining the presence of a specific nucleotide sequence in an RNA reagent of a target sample, said method comprising the steps of:
a) incubating a mixture comprising:
(i) a first component including an RNA reagent extracted directly from a target sample, said RNA reagent having a target nucleotide sequence and a capture sequence, and
(ii) a second component comprising a capture reagent having multiple first arms containing a label capable of emitting a detectable signal and multiple second arms having a nucleotide sequence complementary to the capture sequence of the RNA reagent of the first component,
at a first temperature and for a time sufficient to induce the capture sequence of the RNA reagent of the first component to bind to the complementary nucleotide sequence of the capture reagent of the second component, and thereby form a pre-hybridized RNA- capture reagent complex comprising the target nucleotide sequence;
b) contacting the pre-hybridized RNA-capture reagent complex with a microarray having thereon a plurality of features each containing a particular probe nucleotide sequence;
c) incubating the pre-hybridized RNA-capture reagent complex on the microarray at a second temperature and for a time sufficient to hybridize the target nucleotide sequence of the pre-hybridized RNA-capture reagent complex to the complementary probe nucleotide sequence contained within the feature, and
d) washing said microarray,
wherein the presence of such hybridization results in the emission of the detectable signal from the corresponding feature, and the absence thereof results in no emission of the detectable signal from the corresponding feature, thus generating a detectable hybridization pattern for subsequent analysis, wherein,
after incubation of the first and second components, blocking nucleic acids are added to the mixture of the first and second components, and
said blocking nucleic acids are comprised of short segments complementary to the capture sequence of said first component and to said complementary sequence of said second component, respectively.

2. The method of claim 1 wherein said multiple first arms have a label capable of emitting a detectable signal attached to it.

3. The method of claim 1 or 2 wherein said multiple second arms have a nucleotide sequence complementary to the capture sequence of the RNA reagent of the first component attached to it.

4. The method of any of the preceding claims wherein the RNA reagent and the capture reagent are incubated at the first temperature of from about 45° to 60° C.

5. The method of any one of the preceding claims wherein the pre-hybridized RNA/capture reagent complex is incubated on the microarray at the second temperature of from about 45° to 65° C.

6. The method of any one of the preceding claims wherein the pre-hybridized RNA/capture reagent complex are incubated on the microarray for the sufficient time ranging from about 15 minutes to 24 hours.

7. The method of any one of the preceding claims wherein after incubating the pre-hybridized RNA/recapture reagent complex on the microarray, further comprising washing any free un-hybridized RNA/capture reagent complex from the microarray.

8. A method for determining the presence of a specific nucleotide sequence in an RNA reagent of a target sample, said method comprising the steps of:
a) contacting a first component including an RNA reagent extracted directly from a target sample, said RNA reagent having a target nucleotide sequence and a capture sequence with a microarray having thereon a plurality of features each containing a particular probe nucleotide sequence;
b) incubating the RNA reagent and the complementary probe nucleotide sequences on the microarray at a first temperature and for a time sufficient to hybridize the target nucleotide sequence of the RNA reagent to the complementary probe nucleotide sequence contained within the feature;
c) contacting a second component comprising a capture reagent having multiple first arms containing a label capable of emitting a detectable signal and multiple second arms having a nucleotide sequence complementary to the capture sequence of the RNA reagent of the first component;
d) incubating the capture reagent and the capture sequence of the RNA reagent at a second temperature and for a time sufficient to induce the capture sequence of the RNA reagent of the first component to hybridize to the complementary nucleotide sequence of the capture reagent of the second component, and
e) washing said microarray,
wherein the presence of the hybridization results in the emission of the detectable signal from the corresponding feature, and the absence thereof results in no emission of the detectable signal from the corresponding feature, thus generating a detectable hybridization pattern for subsequent analysis, wherein,
after incubating the capture sequence of the RNA reagent and the capture reagent on the microarray, blocking nucleic acids are added to the microarray, and
said blocking nucleic acids are comprised of short segments complementary to the capture sequence of said first component and to said complementary sequence of said second component, respectively.

9. The method of claim 8, wherein said label capable of emitting a detectable signal is attached to said first arms of the capture reagent.

10. The method of claim 8 or 9 wherein said multiple second arms have a nucleotide sequence complementary to the capture sequence of the RNA reagent of the first component attached to it.

11. The method of any one of claims 1 to 10 wherein the capture reagent is selected from the group consisting of dendrimers, carbohydrates, proteins, and nucleic acids.

12. The method of claim 11 wherein the capture reagent is a dendrimer.

13. The method of any one of the preceding claims further comprising passing a base solution over the microarray to separate and purge the hybridized RNA reagent from the probe nucleotide sequence for enabling reuse of the microarray.

14. The method of claim 13 wherein the base solution is passed over the microarray at a temperature of from about 50° to 60° C.

15. The method of claim 13 or 14 wherein the base solution is 0.05 M sodium hydroxide.

16. The method of any one of the preceding claims wherein the capture sequence of the RNA reagent is a single-stranded oligonucleotide consisting of at least one adenine base.

17. The method of claim 16 wherein the nucleotide sequence complementary to the capture sequence is a single-stranded oligonucleotide consisting of at least one thymine base.

18. The method of any one of the preceding claims wherein the RNA reagent and the complementary probe nucleotide sequences on the microarray are incubated at the first temperature of from about 45° to 65° C.

19. The method of any one of the preceding claims wherein the RNA reagent and the complementary probe nucleotide sequences on the microarray are incubated for a sufficient time ranging from about 15 minutes to 24 hours.

20. The method of any one of the preceding claims wherein the probe nucleotide sequences of the microarray comprises cDNA.

21. The method of any one of the preceding claims wherein the first temperature is from about 60° to 65°C.

22. The method of any one of the preceding claims wherein the probe nucleotide sequences of microarray comprises oligonucleotides.

23. The method of any one of the preceding claims wherein the second temperature is from about 45° to 55°C.

24. The method of any one of the preceding claims wherein the capture regent and the capture sequence of the RNA reagent are incubated at a second temperature ranging from about 45° to 60°C.

25. The method of any one of the preceding claims wherein the capture reagent and the capture sequence of the RNA reagent are incubated for the sufficient time ranging from about 15 minutes to 24 hours.

26. The method of any one of the preceding claims, after the incubating the RNA reagent and the complementary nucleotide probes on the microarray step, further comprising washing the microarray with a buffer solution to remove excess unhybridized RNA reagent.

27. The method of any one of the preceding claims wherein the capture sequence of the RNA reagent comprises at least one locked nucleic acid nucleotide.

## Patentansprüche

1. Verfahren zum Bestimmen des Vorhandenseins einer speziellen Nukleotidsequenz in einem RNA-Reagens einer Zielprobe, wobei das Verfahren die Schritte umfasst:
a) Inkubieren einer Mischung, umfassend:
(i) eine erste Komponente, umfassend ein RNA-Reagens, welches direkt aus einer Zielprobe extrahiert worden ist, wobei das RNA-Reagens eine Ziel-Nukleotidsequenz und eine Einfang-Sequenz aufweist, und
(ii) eine zweite Komponente, umfassend ein Einfang-Reagens mit einer Mehrzahl von ersten Armen, welche eine Markierung, welche in der Lage ist, ein detektierbares Signal zu emittieren, enthalten, und einer Mehrzahl von zweiten Armen, welche eine Nukleotidsequenz aufweisen, welche zu der Einfang-Sequenz des RNA-Reagens der ersten Komponente komplementär ist,
bei einer ersten Temperatur und für eine Zeitspanne, welche ausreichen, um zu induzieren, dass die Einfang-Sequenz des RNA-Reagens der ersten Komponente an die komplementäre Nukleotidsequenz des Einfang-Reagens der zweiten Komponente bindet und dadurch einen vorab hybridisierten RNA-Einfang-Reagens-Komplex, welcher die Ziel-Nukleotidsequenz umfasst, bildet;
b) Inkontaktbringen des vorab hybridisierten RNA-Einfang-Reagens-Komplexes mit einer Mikroanordnung, welche darauf eine Mehrzahl von Merkmalen, von welchen jedes eine bestimmte Sonden-Nukleotidsequenz enthält, aufweist;
c) Inkubieren des vorab hybridisierten RNA-Einfang-Reagens-Komplexes auf der Mikroanordnung bei einer zweiten Temperatur und für eine Zeitspanne, welche ausreichen, um die Ziel-Nukleotidsequenz des vorab hybridisierten RNA-Einfang-Reagens-Komplexes mit der komplementären Sonden-Nukleotidsequenz, die innerhalb des Merkmals enthalten ist, zu hybridisieren, und
d) Waschen der Mikroanordnung,
wobei das Vorhandensein einer solchen Hybridisierung zu der Emission des detektierbaren Signals von dem entsprechenden Merkmal führt und das Fehlen davon zu keiner Emission des detektierbaren Signals von dem entsprechenden Merkmal führt, wodurch ein detektierbares Hybridisierungsmuster für eine nachfolgende Analyse erzeugt wird, wobei
nach dem Inkubieren der ersten und zweiten Komponenten blockierende Nukleinsäuren zu der Mischung der ersten und zweiten Komponenten zugesetzt werden und
wobei die blockierenden Nukleinsäuren kurze Segmente umfassen, welche zu der Einfang-Sequenz der ersten Komponente bzw. zu der komplementären Sequenz der zweiten Komponente komplementär sind.

2. Verfahren nach Anspruch 1, wobei die Mehrzahl von ersten Armen eine Markierung aufweist, welche in der Lage ist, ein detektierbares Signal, welches daran angeheftet ist, zu emittieren.

3. Verfahren nach Anspruch 1 oder 2, wobei die Mehrzahl von zweiten Armen eine Nukleotidsequenz aufweist, welche zu der Einfang-Sequenz des RNA-Reagens der ersten Komponente, das daran angeheftet ist, komplementär ist.

4. Verfahren nach einem der vorangegangenen Ansprüche, wobei das RNA-Reagens und das Einfang-Reagens bei der ersten Temperatur von etwa 45° bis 60°C inkubiert werden.

5. Verfahren nach einem der vorangegangenen Ansprüche, wobei der vorab hybridisierte RNA/Einfang-Reagens-Komplex auf der Mikroanordnung bei der zweiten Temperatur von etwa 45° bis 65°C inkubiert wird.

6. Verfahren nach einem der vorangegangenen Ansprüche, wobei der vorab hybridisierte RNA/Einfang-Reagens-Komplex auf der Mikroanordnung für die ausreichende Zeitspanne, welche von etwa 15 Minuten bis 24 Stunden reicht, inkubiert wird.

7. Verfahren nach einem der vorangegangenen Ansprüche, wobei dieses nach dem Inkubieren des vorab hybridisierten RNA/erneuter Einfang-Reagens-Komplexes auf der Mikroanordnung weiter umfasst, jeglichen nicht-hybridisierten RNA/Einfang-Reagens-Komplex von der Mikroanordnung fortzuwaschen.

8. Verfahren zum Bestimmen des Vorhandenseins einer speziellen Nukleotidsequenz in einem RNA-Reagens einer Zielprobe, wobei das Verfahren die Schritte umfasst:
a) Inkontaktbringen einer ersten Komponente, welche ein direkt aus einer Zielprobe extrahiertes RNA-Reagens umfasst, wobei das RNA-Reagens eine Ziel-Nukleotidsequenz und eine Einfang-Sequenz aufweist, mit einer Mikroanordnung, welche darauf eine Mehrzahl von Merkmalen, welche jeweils eine bestimmte Sonden-Nukleotidsequenz enthalten, aufweist;
b) Inkubieren des RNA-Reagens und der komplementären Sonden-Nukleotidsequenzen auf der Mikroanordnung bei einer ersten Temperatur und für eine Zeitspanne, welche ausreichen, um die Ziel-Nukleotidsequenz des RNA-Reagens mit der komplementären Sonden-Nukleotidsequenz, die innerhalb des Merkmals enthalten ist, zu hybridisieren;
c) Inkontaktbringen einer zweiten Komponente, umfassend ein Einfang-Reagens mit einer Mehrzahl von ersten Armen, welche eine Markierung enthalten, welche in der Lage ist, ein detektierbares Signal zu emittieren, und einer Mehrzahl von zweiten Armen, welche eine Nukleotidsequenz aufweisen, welche komplementär zu der Einfang-Sequenz des RNA-Reagens der ersten Komponente ist;
d) Inkubieren des Einfang-Reagens und der Einfang-Sequenz des RNA-Reagens bei einer zweiten Temperatur und für eine Zeitspanne, welche ausreichen, um zu induzieren, dass die Einfang-Sequenz des RNA-Reagens der ersten Komponente mit der komplementären Nukleotidsequenz des Einfang-Reagens der zweiten Komponente hybridisiert; und
e) Waschen der Mikroanordnung,
wobei das Vorhandensein der Hybridisierung zu der Emission des detektierbaren Signals von dem entsprechenden Merkmal führt und das Fehlen davon zu keiner Emission des detektierbaren Signals von dem entsprechenden Merkmal führt, wodurch ein detektierbares Hybridisierungsmuster für eine nachfolgende Analyse erzeugt wird, wobei
nach dem Inkubieren der Einfang-Sequenz des RNA-Reagens und des Einfang-Reagens auf der Mikroanordnung blockierende Nukleinsäuren zu der Mikroanordnung zugesetzt werden und
wobei die blockierenden Nukleinsäuren kurze Segmente umfassen, welche zu der Einfang-Sequenz der ersten Komponente bzw. zu der komplementären Sequenz der zweiten Komponente komplementär sind.

9. Verfahren nach Anspruch 8, wobei die Markierung, welche in der Lage ist, ein detektierbares Signal zu emittieren, an die ersten Arme des Einfang-Reagens angeheftet ist.

10. Verfahren nach Anspruch 8 oder 9, wobei die Mehrzahl von zweiten Armen eine Nukleotidsequenz aufweist, welche zu der Einfang-Sequenz des RNA-Reagens der ersten Komponente, das daran angeheftet ist, komplementär ist.

11. Verfahren nach einem der Ansprüche 1 bis 10, wobei das Einfang-Reagens aus der Gruppe bestehend aus Dendrimeren, Kohlenhydraten, Proteinen und Nukleinsäuren ausgewählt ist.

12. Verfahren nach Anspruch 11, wobei das Einfang-Reagens ein Dendrimer ist.

13. Verfahren nach einem der vorangegangenen Ansprüche, welches weiter umfasst, eine Basenlösung über die Mikroanordnung zu leiten, um das hybridisierte RNA-Reagens von der Sonden-Nukleotidsequenz abzulösen und wegzuwaschen, um eine Wiederverwendung der Mikroanordnung zu ermöglichen.

14. Verfahren nach Anspruch 13, wobei die Basenlösung über die Mikroanordnung bei einer Temperatur von etwa 50° bis 60°C geleitet wird.

15. Verfahren nach Anspruch 13 oder 14, wobei die Basenlösung 0,05 M Natriumhydroxid ist.

16. Verfahren nach einem der vorangegangenen Ansprüche, wobei die Einfang-Sequenz des RNA-Reagens ein einzelsträngiges Oligonukleotid bestehend aus mindestens einer Adenin-Base ist.

17. Verfahren nach Anspruch 16, wobei die Nukleotidsequenz, die zu der Einfang-Sequenz komplementär ist, ein einzelsträngiges Oligonukleotid bestehend aus mindestens einer Thymin-Base ist.

18. Verfahren nach einem der vorangegangenen Ansprüche, wobei das RNA-Reagens und die komplementären Sonden-Nukleotidsequenzen auf der Mikroanordnung bei der ersten Temperatur von etwa 45° bis 65°C inkubiert werden.

19. Verfahren nach einem der vorangegangenen Ansprüche, wobei das RNA-Reagens und die komplementären Sonden-Nukleotidsequenzen auf der Mikroanordnung für eine ausreichende Zeitspanne, welche von etwa 15 Minuten bis 24 Stunden reicht, inkubiert werden.

20. Verfahren nach einem der vorangegangenen Ansprüche, wobei die Sonden-Nukleotidsequenzen der Mikroanordnung cDNA umfassen.

21. Verfahren nach einem der vorangegangenen Ansprüche, wobei die erste Temperatur etwa 60° bis 65°C beträgt.

22. Verfahren nach einem der vorangegangenen Ansprüche, wobei die Sonden-Nukleotidsequenzen der Mikroanordnung Oligonukleotide umfassen.

23. Verfahren nach einem der vorangegangenen Ansprüche, wobei die zweite Temperatur etwa 45° bis 55°C beträgt.

24. Verfahren nach einem der vorangegangenen Ansprüche, wobei das Einfang-Reagens und die Einfang-Sequenz des RNA-Reagens bei einer zweiten Temperatur, welche von etwa 45° bis 60°C reicht, inkubiert werden.

25. Verfahren nach einem der vorangegangenen Ansprüche, wobei das Einfang-Reagens und die Einfang-Sequenz des RNA-Reagens für die ausreichende Zeitspanne, welche von etwa 15 Minuten bis 24 Stunden reicht, inkubiert werden.

26. Verfahren nach einem der vorangegangenen Ansprüche, welches nach dem Schritt des Inkubierens des RNA-Reagens und der komplementären Nukleotidsonden auf der Mikroanordnung weiter umfasst, die Mikroanordnung mit einer Pufferlösung zu waschen, um überschüssiges nicht-hybridisiertes RNA-Reagens zu entfernen.

27. Verfahren nach einem der vorangegangenen Ansprüche, wobei die Einfang-Sequenz des RNA-Reagens wenigstens ein eingeschlossenes bzw. locked Nukleinsäure-Nukleotid umfasst.

## Revendications

1. Procédé pour déterminer la présence d'une séquence nucléotidique spécifique dans un réactif ARN d'un échantillon cible, ledit procédé comprenant les étapes de :
a) incuber un mélange comprenant :
(i) un premier composant incluant un réactif ARN extrait directement d'un échantillon cible, ledit réactif ARN ayant une séquence nucléotidique cible et une séquence de capture, et
(ii) un second composant comprenant un réactif de capture ayant de multiples premiers bras contenant un marqueur capable d'émettre un signal détectable et de multiples seconds bras ayant une séquence nucléotidique complémentaire de la séquence de capture du réactif ARN du premier composant,
à une première température et pendant une durée suffisante pour amener la séquence de capture du réactif ARN du premier composant à se lier à la séquence nucléotidique complémentaire du réactif de capture du second composant, et former ainsi un complexe ARN-réactif de capture préhybridé comprenant la séquence nucléotidique cible ;
b) mettre en contact le complexe ARN-réactif de capture préhybridé avec une micromatrice ayant sur elle une pluralité de caractéristiques contenant chacune une séquence nucléotidique sonde particulière ;
c) incuber le complexe ARN-réactif de capture préhybridé sur la micromatrice à une seconde température et pendant une durée suffisante pour hybrider la séquence nucléotidique cible du complexe ARN-réactif de capture préhybridé à la séquence nucléotidique sonde complémentaire contenue dans la caractéristique, et
d) laver ladite micromatrice,
où la présence d'une telle hybridation conduit à l'émission du signal détectable depuis la caractéristique correspondante, et son absence conduit à l'absence d'émission du signal détectable depuis la caractéristique correspondante, en produisant ainsi un motif d'hybridation détectable pour une analyse subséquente, où,
après l'incubation des premier et second composants, des acides nucléiques bloquants sont ajoutés au mélange des premier et second composants, et
lesdits acides nucléiques bloquants comprennent de courts segments complémentaires de la séquence de capture dudit premier composant et de ladite séquence complémentaire dudit second composant, respectivement.

2. Procédé selon la revendication 1 où lesdits premiers bras multiples ont un marqueur capable d'émettre un signal détectable fixé à lui.

3. Procédé selon la revendication 1 ou 2 où lesdits seconds bras multiples ont une séquence nucléotidique complémentaire de la séquence de capture du réactif ARN du premier composant fixée à lui.

4. Procédé selon l'une quelconque des revendications précédentes où le réactif ARN et le réactif de capture sont incubés à la première température d'environ 45° à 60°C.

5. Procédé selon l'une quelconque des revendications précédentes où le complexe ARN/réactif de capture préhybridé est incubé sur la micromatrice à la seconde température d'environ 45° à 65°C.

6. Procédé selon l'une quelconque des revendications précédentes où le complexe ARN/réactif de capture préhybridé est incubé sur la micromatrice pendant la durée suffisante allant d'environ 15 min à 24 h.

7. Procédé selon l'une quelconque des revendications précédentes où, après l'incubation du complexe ARN/réactif de capture préhybridé sur la micromatrice, comprenant en outre le retrait par lavage de tout complexe ARN/réactif de capture non hybridé libre de la micromatrice.

8. Procédé pour déterminer la présence d'une séquence nucléotidique spécifique dans un réactif ARN d'un échantillon cible, ledit procédé comprenant les étapes de :
a) mettre en contact un premier composant incluant un réactif ARN extrait directement d'un échantillon cible, ledit réactif ARN ayant une séquence nucléotidique cible et une séquence de capture avec une micromatrice ayant sur elle une pluralité de caractéristiques contenant chacune une séquence nucléotidique sonde particulière ;
b) incuber le réactif ARN et les séquences nucléotidiques sondes complémentaires sur la micromatrice à une première température et pendant une durée suffisante pour hybrider la séquence nucléotidique cible du réactif ARN à la séquence nucléotidique sonde complémentaire contenue dans la caractéristique ;
c) mettre en contact un second composant comprenant un réactif de capture ayant de multiples premiers bras contenant un marqueur capable d'émettre un signal détectable et de multiples seconds bras ayant une séquence nucléotidique complémentaire de la séquence de capture du réactif ARN du premier composant ;
d) incuber le réactif de capture et la séquence de capture du réactif ARN à une seconde température et pendant une durée suffisante pour amener la séquence de capture du réactif ARN du premier composant à s'hybrider à la séquence nucléotidique complémentaire du réactif de capture du second composant, et
e) laver ladite micromatrice,
où la présence de l'hybridation conduit à l'émission du signal détectable depuis la caractéristique correspondante, et son absence conduit à l'absence d'émission du signal détectable depuis la caractéristique correspondante, en produisant ainsi un motif d'hybridation détectable pour une analyse subséquente, où,
après l'incubation de la séquence de capture du réactif ARN et du réactif de capture sur la micromatrice, des acides nucléiques bloquants sont ajoutés à la micromatrice, et
lesdits acides nucléiques bloquants comprennent de courts segments complémentaires de la séquence de capture dudit premier composant et de ladite séquence complémentaire dudit second composant, respectivement.

9. Procédé selon la revendication 8 où ledit marqueur capable d'émettre un signal détectable est fixé audits premiers bras du réactif de capture.

10. Procédé selon la revendication 8 ou 9 où lesdits seconds bras multiples ont une séquence nucléotidique complémentaire de la séquence de capture du réactif ARN du premier composant fixée à lui.

11. Procédé selon l'une quelconque des revendications 1 à 10
où le réactif de capture est choisi dans le groupe consistant en les dendrimères, les glucides, les protéines et les acides nucléiques.

12. Procédé selon la revendication 11 où le réactif de capture est un dendrimère.

13. Procédé selon l'une quelconque des revendications précédentes comprenant en outre le passage d'une solution basique sur la micromatrice pour séparer et retirer le réactif d'ARN hybridé de la séquence nucléotidique sonde pour permettre une réutilisation de la micromatrice.

14. Procédé selon la revendication 13 où la solution basique est amenée à passer sur la micromatrice à une température d'environ 50° à 60°C.

15. Procédé selon la revendication 13 ou 14 où la solution basique est de l'hydroxyde de sodium 0,05 M.

16. Procédé selon l'une quelconque des revendications précédentes où la séquence de capture du réactif ARN est un oligonucléotide simple brin consistant en au moins une base adénine.

17. Procédé selon la revendication 16 où la séquence nucléotidique complémentaire de la séquence de capture est un oligonucléotide simple brin consistant en au moins une base thymine.

18. Procédé selon l'une quelconque des revendications précédentes où le réactif ARN et les séquences nucléotidiques sondes complémentaires sur la micromatrice sont incubés à la première température d'environ 45° à 65°C.

19. Procédé selon l'une quelconque des revendications précédentes où le réactif ARN et les séquences nucléotidiques sondes complémentaires sur la micromatrice sont incubés pendant une durée suffisante allant d'environ 15 min à 24 h.

20. Procédé selon l'une quelconque des revendications précédentes où les séquences nucléotidiques sondes de la micromatrice comprennent un ADNc.

21. Procédé selon l'une quelconque des revendications précédentes où la première température est d'environ 60° à 65°C.

22. Procédé selon l'une quelconque des revendications précédentes où les séquences nucléotidiques sondes de la micromatrice comprennent des oligonucléotides.

23. Procédé selon l'une quelconque des revendications précédentes où la seconde température est d'environ 45° à 55°C.

24. Procédé selon l'une quelconque des revendications précédentes où le réactif de capture et la séquence de capture du réactif ARN sont incubés à une seconde température allant d'environ 45° à 60°C.

25. Procédé selon l'une quelconque des revendications précédentes où le réactif de capture et la séquence de capture du réactif ARN sont incubés pendant la durée suffisante allant d'environ 15 min à 24 h.

26. Procédé selon l'une quelconque des revendications précédentes, après l'étape d'incubation du réactif ARN et des sondes nucléotidiques complémentaires sur la micromatrice, comprenant en outre le lavage de la micromatrice avec une solution tampon pour retirer le réactif ARN non hybridé en excès.

27. Procédé selon l'une quelconque des revendications précédentes où la séquence de capture du réactif ARN comprend au moins un nucléotide d'acide nucléique bloqué.
